# EUROPÄISCHE PATENTSCHRIFT

(11) **EP 1 863 441 B1**
(45) Veröffentlichungstag und Bekanntmachung des Hinweises auf die Patenterteilung: **21.11.2012**
(21) Anmeldenummer: 06708336.0
(22) Anmeldetag: 17.02.2006
(51) Int. Cl.: A61K 8/81, A61Q 19/10, A61Q 5/02, A61Q 5/12

(54) **PFLEGESYSTEM AUS PVP-UND ACRYLATPOLYMEREN**
CARE SYSTEM CONSTITUTED OF PVP AND ACRYLATE POLYMERS
SYSTEME DE SOINS A BASE DE POLYMERES DE POLYVINYLPYRROLIDONE ET D'ACRYLATE

(30) Priorität: 24.03.2005 DE 102005014423
(43) Veröffentlichungstag der Anmeldung: 12.12.2007
(73) Patentinhaber: Beiersdorf Aktiengesellschaft, 20253 Hamburg (DE)
(72) Erfinder: DEMITZ, Michael, 22529 Hamburg (DE); DOERSCHNER, Albrecht, 20146 Hamburg (DE); KOHUT, Michaela, 20255 Hamburg (DE); RUPPERT, Stephan, 20259 Hamburg (DE); ALBRECHT, Harald, 22083 Hamburg (DE); KUETHER, Joerg, 25469 Halstenbek (DE); HEITMANN, Birgit, 22769 Hamburg (DE)
(86) Internationale Anmeldenummer: PCT/EP2006/060045
(87) Internationale Veröffentlichungsnummer: WO 2006/100161

(56) Entgegenhaltungen:
- EP-A- 0 636 357
- WO-A-01/19946
- WO-A-01/76552
- US-A- 4 676 978

## Beschreibung

Die vorliegende Erfindung betrifft kosmetische Reinigungsmittel mit besonderer Pflegeleistung. Kosmetische Reinigungsmittel sind an sich bekannt. Es handelt sich dabei im wesentlichen um oberflächenaktive Substanzen oder Stoffgemische, die dem Verbraucher in verschiedenen Zubereitungen angeboten werden.

Unter Pflegeleistung im Sinne der vorliegenden Schrift versteht man bei Körperreinigungsmitteln das Erreichen einer sehr guten Reinigungsleistung bei gleichzeitig hervorragender Milde und Produktverträglichkeit. Dem Verbraucher soll es ermöglicht werden entsprechende Produkte auch mehrmals täglich anwenden zu können, ohne Beeinträchtigungen der Hautgesundheit befürchtet zu müssen.

Unter Pflegeleistung im Sinne der vorliegenden Schrift versteht man bei Haarreinigungsmitteln das Vermögen der Zusammensetzung beim Benutzer derselben sauberes Haar, eine gute Kämmbarkeit des Haares, im nassen und trockenen Zustand, eine gute Frisierbarkeit, einen guten Griff und einen guten Haarglanz zu erzielen.

Zubereitungen dieser Art sind beispielsweise Schaum- und Duschbäder, feste und flüssige Seifen oder sogenannte "Syndets" (synthetische Detergentien), Shampoos, Handwaschpasten, Intimwaschmittel, spezielle Reinigungsmittel für Kleinkinder und Babys und dergleichen.

Oberflächenaktive Stoffe - am bekanntesten die Alkalisalze der höheren Fettsäuren, also die klassischen "Seifen" - sind amphiphile Stoffe, die organische unpolare Substanzen in Wasser emulgieren können.

Diese Stoffe schwemmen nicht nur Schmutz von Haut und Haaren, sie reizen, je nach Wahl des Tensids oder des Tensidgemisches, Haut und Schleimhäute mehr oder minder stark.

Das gebräuchlichste Tensid für kosmetische Zusammensetzungen ist das Natriumlaurylethersulfat. Obwohl es gute Waschkraft besitzt und gut haut- und schleimhautverträglich ist, sollten empfindliche Personen den häufigen Kontakt damit meiden.

Es ist zwar eine große Zahl recht milder Tenside erhältlich, jedoch sind die Tenside des Standes der Technik entweder mild, reinigen aber schlecht, oder aber sie reinigen gut, reizen jedoch Haut oder Schleimhäute.

Diesem Übelstande galt es also, Abhilfe zu schaffen.

Die vorliegende Erfindung betrifft in einer besonderen Ausführungsform Reinigungszubereitungen für die Verwendung als Duschpräparat.

Auch derartige Zubereitungen sind an sich bekannt. Es handelt sich dabei im wesentlichen um oberflächenaktive Substanzen oder Stoffgemische, die dem Verbraucher in verschiedenen Zubereitungen angeboten werden. Zubereitungen solcher Art zeichnen sich im allgemeinen durch einen mehr oder weniger hohen Wassergehalt aus, können aber auch beispielsweise als Konzentrat vorliegen.

Im allgemeinen unterscheiden sich Präparate, weiche für das Duschbad vorgesehen sind, nicht oder kaum von Wannenbadzubereitungen, abgesehen davon, dass bei Duschzubereitungen Produkte höherer Viskosität bevorzugt werden, die nicht nach Entnahme aus dem Behälter aus der Hand rinnen. Dies ist bei Wannenbadzubereitungen weniger von praktischer Bedeutung.

Schon bei einem einfachen Wasserbade ohne Zusatz von Tensiden kommt es zunächst zu einer Quellung der Hornschicht der Haut, wobei der Grad dieser Quellung beispielsweise von der Dauer des Bades und dessen Temperatur abhängt. Zugleich werden wasserlösliche Stoffe, z.B. wasserlösliche Schmutzbestandteile, aber auch hauteigene Stoffe, die für das Wasserbindungsvermögen der Hornschicht verantwortlich sind, ab- bzw. ausgewaschen. Durch hauteigene oberflächenaktive Stoffe werden zudem auch Hautfette in gewissem Ausmaße gelöst und ausgewaschen. Dies bedingt nach anfänglicher Quellung eine nachfolgende deutliche Austrocknung der Haut, die durch waschaktive Zusätze nach verstärkt werden kann.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Falle nichtpathologischer Abweichungen vom Normalstatus, z.B. durch umweltbedingte Abnutzungsschäden bzw. Irritationen, Lichtschäden, Altershaut usw., ist der Schutzmechanismus der Hautoberfläche gestört. Unter Umständen ist er dann aus eigener Kraft nicht mehr imstande, seine Aufgabe zu erfüllen und muß durch externe Maßnahmen regeneriert werden.

Aufgabe der vorliegenden Erfindung war somit, diesem Mangel des Standes der Technik Abhilfe zu schaffen. Weiterhin war eine Aufgabe der Erfindung, Wannen- aber auch Duschbadzubereitungen zur Verfügung zu stellen, welche einesteils hohe Pflegewirkung besitzen, ohne dass andererseits die reinigende Wirkung dahinter zurücksteht.

Die vorliegende Erfindung betrifft ferner waschaktive haarkosmetische Zubereitungen, landläufig als Shampoos bezeichnet. Insbesondere betrifft die vorliegende Erfindung haarkosmetische Wirkstoffkombinationen und Zubereitungen zur Pflege des Haars und der Kopfhaut.

Auch die Haarwäsche mit aggressiven Tensiden kann das Haar beanspruchen, zumindest dessen Erscheinungsbild oder das Erscheinungsbild der Haartracht insgesamt herabsetzen. Beispielsweise können bestimmte wasserlösliche Haarbestandteile (z.B. Harnstoff, Harnsäure, Xanthin, Keratin, Glycogen, Citronensäure, Milchsäure) durch die Haarwäsche herausgelaugt werden.

Der Stande der Technik ließ es aber an Shampooformulierungen mangeln, welche dem geschädigten Haar in befriedigender Weise Pflege zukommen ließen. Aufgabe war daher, auch diesen Nachteilen des Standes der Technik Abhilfe zu schaffen.

Übliche, und sich gerade in neuerer Zeit immer weiter verbreitende kosmetische und dermatologische Zubereitungsformen sind Gele.

Kosmetische Gele erfreuen sich beim Verbraucher äußerster Beliebtheit. Da sie meistens durchsichtig sind, oftmals eingefärbt aber ebensooft farblos klar sein dürften, bieten sie dem kosmetischen Entwickler zusätzliche Gestaltungsmöglichkeiten, die teilweise funktionalen Charakter haben, teilweise aber auch lediglich der Aufbesserung des äußeren Erscheinungsbildes dienen. So können beispielsweise dem Produkt, welches sich dem Betrachter dann in der Regel in einer durchsichtigen Verpackung darbietet, durch eingearbeitete Farbpigmente, Gasbläschen und dergleichen, oder aber auch größere Objekte, interessante optische Effekte verliehen werden.

Gerade dann, wenn es erwünscht ist, dass das oder die eingearbeiteten Objekte, mögen diese als solche mit dem bloßen Auge als solche erkenntlich sein, mögen sie in mikroskopischen Ausmaßen, aber in interessanter Anordnung - beispielsweise in Form von künstlich erzeugten Farbschlieren - dann doch sichtbare Formen ergeben, so ist es doch wünschenswert, dass diese Objekte in der Gelformulierung ortsfest bleiben und nicht zu Boden sinken oder in irgendeiner Weise in der Formulierungen andere unliebsame Wanderungen vornehmen.

Flüssigkeiten können bezüglich ihrer rheologischen Eigenschaften durch ihr Fließ- und Deformationsverhalten unterschieden werden. Ideal elastische Körper erleiden durch äußere Kräfte eine elastische Deformation, die bei Wegnahme der äußeren Krafteinwirkung ein spontanes, vollständiges Zurückgehen der Deformation bewirkt. Ideal viskose Körper werden durch äußere Kräfte irreversibel in ihrer Form verändert. Die zunehmende Deformation wird als Fließen bezeichnet. Die meisten Flüssigkeiten sind weder ideal viskos noch ideal elastisch, sondern zeigen sowohl viskose als auch elastische Eigenschaften und werden daher als viskoelastische Substanzen bezeichnet.

Im Großteil viskoelastischer Lösungen werden dispergierte Partikel oder Gasbläschen immer sedimentieren bzw. aufsteigen. Sie besitzen eine endliche Strukturrelaxationszeit. Das bedeutet, dass die Netzwerke in diesen Systemen auf eine Deformation mit einer entsprechenden Schubspannung reagieren. Diese wird aber in einer endlichen Zeit auf den Wert Null relaxieren, so dass sich die gesamte Lösung wieder in einem stabilen Ruhezustand ohne Spannung befindet. Dies bedeutet weiter, dass diese Lösungen eine definierte Nullviskosität besitzen und somit bei kleinen Scherraten einen konstanten Viskositätswert erreichen.

Im Gegensatz zu diesen Systemen gibt es aber auch solche, in denen dispergierte Partikel oder Gasbläschen nicht sedimentieren. Es fällt auf, dass diese Systeme erst oberhalb eines charakteristischen Werts fließen. Dieser Wert wird Fließgrenze genannt. Bei näherer Betrachtung der rheologischen Eigenschaften dieser Systeme fällt auf, dass der Speichermodul in ganzen Frequenzbereich unabhängig ist von der Oszillationsfrequenz und immer wesentlich größter ist als der Veriustmodul.

Dagegen erreicht der Betrag der komplexen Viskosität auch bei den kleinsten Frequenzen keinen konstanten Wert, sondern steigt weiter an.

Carbopolgele sind quervernetzte Acrylsäurepolymere, die eine hohe Anzahl von Carboxylgruppen tragen. In gelöster Form binden diese Strukturen Wasser. Die Neutralisation der Carboxylgruppen führt aufgrund deren elektrostatischen Abstoßung zu einer Ausdehnung und damit Quellung der Polymerketten. In diesem Zustand erreichen die Carbopol Gele ihre typischen rheologischen Eigenschaften wie z.B. die Ausbildung einer Fließgrenze.

Der Effekt der Ausbildung einer Fließgrenze beruht somit auf der elektrostatischen Abstoßung der Carboxylgruppen. Zusätzliche Elektrolyte schirmen diese Ladungen ab.

Dadurch kollabieren die Netzwerke, die Fließgrenze bricht zusammen, Partikel oder Gasbläschen können nicht mehr in Schwebe gehalten werden.

Tenside wirken wie Elektrolyte. Daher war es bisher nicht möglich, gut schäumende Reinigungsprodukte mit einem entsprechend hohem Gehalt an Tensid zu formulieren, die klare Carbopol Gele mit Fließgrenze als Basis enthielten.

Der Stand der Technik kennt zwar bereits entsprechende Systeme mit Xanthan Gum (z.B. EP-A 738 509). Diese besitzen aber bezüglich des Hautgefühls während und nach der Anwendung schlechtere kosmetische Eigenschaften. Darüber hinaus können bei gleicher Einsatzkonzentration nur geringere Viskositäten erreicht werden. Die Ausgestaltung eines Gels, welche dazu geeignete Fließeigenschaften aufweist, bietet dem Fachmanne in der Regel keine überaus großen Schwierigkeiten, außer, wenn hohe Tensidkonzentrationen erreicht werden sollen - in der Regel eine Grundanforderung an Reinigungsprodukte. Der Nachteil solch hoher Tensidkonzentrationen ist, dass meistens nur eingetrübte, trübe oder gar opake Produkte erlangt werden.

In der WO 01/19946 werden waschaktive Rezepturen offenbart, die neben einem Gelbildner einen Konditionierer enthalten. In der WO 01 /176552 werden waschaktive Rezepturen offenbart, welche eine Kombination bestimmter Verdicker mit Acylglutamaten betrifft. Diese Schriften konnten jedoch nicht den Weg zur vorliegenden Erfindung weisen.

Die Konsistenz des Produkts beeinflusst die Schaummenge und die Schaumcremigkeit, insbesondere zum Zeitpunkt der Schaumentwicklung und damit die Akzeptanz eines Produkts beim Verbraucher. Bereits bei der Produktverteilung und der Schaumentwicklung in der Hand oder in den Haaren bewertet der Verbraucher das Produkt hinsichtlich der zu erwartenden Pflegeleistung. Eine hohe Konsistenz und eine cremige, reichhaltige Schaumentwicklung ist hierbei gleichbedeutend mit der Erwartungshaltung der reichhaltigen Pflegeleistung.

Um in eine üblichen Shampooformulierung, Duschgel- oder Gesichtreinigungsformulierung eine erhöhte Konsistenz zu erreichen können die üblichen Verdickungsmittel eingesetzt werden. Diese führen jedoch nicht zu einem akzeptablen, kosmetischen Fließ- und Entnahmeverhalten. Vorteilhafter ist es hier spezielle Verdickungssysteme auf der Basis von Polyacrylaten einzusetzen. Mit diesen Systemen lassen sich sogar tensidhaltige Formulierungen (Shampoos) mit Fließgrenzen aufbauen, so dass kleine, sichtbare Partikel in den Produkten in der Schwebe gehalten werden können. Doch diese Systeme führen nicht zu Shampoos mit akzeptabler Pflegeleistung bzw. Duschgelen und Gesichtsreinigem mit ausreichender Hautpflegeleistung bzw. Sensorik. Auch durch den Zusatz üblicher Konditionierstoffe, wie kationischen Polymeren (auch polymeren Quats genannt) kann die Haut- und Haarpflegeleistung, insbesondere die Kämmbarkeit im nassen und trockenen Haar, nicht auf ein für trocken/strapaziertes Haar gewünschtes Shampoo eingestellt werden.
Eine weitere Aufgabe der vorliegenden Erfindung war es daher, Wege zu finden, die es erlauben, polyacrylathaltige Shampoos mit kationischen Polymeren bei gleichzeitig vergleichsweise guter Pflegeleistung herzustellen.

Auch diesem Nachteil des Standes der Technik galt es also, Abhilfe zu schaffen.

EP 0636357 offenbart Haarbehandlungsmittel, welche Kombinationen aus amphoteren Tensiden, kationischen Tensiden und PVP enthalten. In den erfindungsgemäßen Zubereitungen kann auf den Einsatz von kationischen Tensiden verzichtet werden, um die gewünschte Pflegeleistung zu erzielen.

EP 200620 offenbart Haarbehandlungsmittel, enthaltend ethoxylierte Triglyceride, PVP und Phenyltrimethicon. In den erfindungsgemäßen Zubereitungen kann auf den Einsatz von Phenyltrimethicon verzichtet werden, um die gewünschte Pflegeleistung zu erzielen.

DE 3336760 offenbart Reinigungszubereitungen, enthaltend Alkylpolyglucoside und PVP. In den erfindungsgemäßen Zubereitungen kann auf den Einsatz von Alkylpolyglukosiden verzichtet werden, um die gewünschte Pflegeleistung zu erzielen.

US 4676978 offenbart Haarreinigungszusammensetrungen, enthaltend Laurylethersulfat, Guar Quat, Polyethylenglykolpolyamin und PVP. In den erfindungsgemäßen Zubereitungen kann auf den Einsatz von Polyethylenglykolpolymain verzichtet werden, um die gewünschte Pflegeleistung zu erzielen.

WO 02058646 offenbart Haarbehandlungsmittel, enthaltend einen Schutzwirkstoff, Konditioniermittel und Copolymere mit PVP-Einheiten. In den erfindungsgemäßen Zubereitungen kann auf den Einsatz dieser speziellen Copolymere verzichtet werden, um die gewünschte Pflegeleistung zu erzielen.

WO 02/65996 offenbart Zubereitungen mit zwei verschiedenen anionischen Assoziativpolymeren, die jedoch nicht erfindungsgemäße Pflegeleistung auf Haaren bzw. der Haut erreichen.

US 2002/42448 offenbart ein System zur Verdickung und rheologischen Einstellung mit einem komplizierten Terpolymer und einem Polysaccharid, welches jedoch nicht die erfindungsgemäße Pflegeleistung auf Haaren bzw. der Haut erreicht.

DE 3414090 offenbart Zubereitungen mit einem anionischen Polymer und spezieller oberflächenaktive Agentien, welche jedoch nicht die erfindungsgemäße Pflegeleistung auf Haaren bzw. der Haut erreichen.

EP 1090631 offenbart Zubereitungen mit einem Verdicker und einem Transparenzmittel (Polyole). In den erfindungsgemäßen Zubereitungen kann auf den Einsatz von Polyolen verzichtet werden.

EP 965322 offenbart tensidhaltige Zubereitungen mit bestimmten Polymeren in Kombination mit ethoxylierten Alkoholen, die jedoch nichts mit den erfindungsgemäßen Polymerkombinationen gemein haben. In den erfindungsgemäßen Zubereitungen kann auf den Einsatz von ethoxylierten Alkoholen verzichtet werden

EP 1010422 offenbart tensidhaltige Zubereitungen mit zwei verschiedenen Verdickersystemen, die aus Polyacrylat und Polyalkoxyestern aufgebaut sind. In den erfindungsgemäßen Zubereitungen kann auf den Einsatz von Polyalkoxyestern verzichtet werden.

EP 1291001 offenbart Zubereitungen mit bestimmten Acrylatcopolymeren und Ölen in Konzentratione von größer 0,2%. In den erfindungsgemäßen Zubereitungen kann auf den Einsatz dieser Öle in Konzentrationen von größer oder gleich 0,2% verzichtet werden, um die gewünschte Pflegeleistung zu erhalten.

Es hat sich überraschend gezeigt, und darin liegt die Lösung dieser Aufgaben, dass kosmetische und dermatologische waschaktive Zubereitungen zur Reinigung der Haut und/oder zur Haar- und Kopfhautreinigung, enthaltend
(a) eine wirksame Menge an einem oder mehreren anionischen Tensiden,
(b) eine wirksame Menge an einem oder mehreren amphotheren Tensiden,
(c) eine wirksame Menge eines oder mehrerer gelbildender Acrylatverdicker gewählt aus der Gruppe der vernetzten alkali-quellbaren Acrylat Copolymere,
(d) ein Homo- oder Copolymer auf der Basis von Vinylpyrrolidon, besonders bevorzugt Polyvinylpyrrolidon,
(e) gewünschtenfalls weitere übliche Hilfs- und/oder Zusatzstoffe, insbesondere Wasser,
den Nachteilen des Standes der Technik abhelfen.

Dabei ist es von Vorteil, wenn die Formulierungen zusätzlich kationische Polymere enthalten.

Dabei ist es von Vorteil, wenn als anionisches Tensid Laurylethersulfat und/oder Myrethethersulfate und als amphotheres Tensid Cocoamidopropylbetain gewählt wird.

Dabei ist es von Vorteil, wenn die Gesamtmenge an einer oder mehreren erfindungsgemäß verwendeten Tenside aus dem Bereich von 0,1 -25 Gew.%, bevorzugt 1 - 12 Gew.-%, insbesondere bevorzugt 2 - 10 Gew.-% gewählt wird, jeweils bezogen auf das Gesamtgewicht der Zubereitungen.

Dabei ist es von Vorteil, wenn die Gesamtmenge an einer oder mehreren erfindungsgemäß verwendeten anionischen Tenside aus dem Bereich von 5 bis 15 Gew.-%, bevorzugt 6 bis 12 Gew.-% gewählt wird, jeweils bezogen auf das Gesamtgewicht der Zubereitungen.

Dabei ist es von Vorteil, wenn die Gesamtmenge an einer oder mehreren erfindungsgemäß verwendeten amphotheren Tenside aus dem Bereich von 0,5 bis 10 Gew.-%, bevorzugt 1 bis 5 Gew.-% gewählt wird, jeweils bezogen auf das Gesamtgewicht der Zubereitungen.

Dabei ist es von Vorteil, wenn als Acrylatverdicker ein Copolymer verwendet wird, bestehend aus a) einem Acrylatmonomer ausgewählt aus Acrylsäure, Methacrylsäure, Itaconsäure, Fumarsäure, Crotonsäure, Aconitsäure oder Maleinsäure, b) einem a,b-ethylenisch ungesättigten Monomer der allgemeinen Formel CH₂=CXY mit X=H, CH₃, - C1-C30-Alkyl, -CH₂-C(=O)O(CH₂-CH₂O)ₓ-R³, -CH₂C(=O)NH(CO₂-CH₂Oₓ-R³, -CH₂-CH₂=(CH₂-CH₂O)ₓ-R₃ mit x= 1-100 und R³=C1-C30 Alkyl oder Cl und Y= -COOR, - C₆H₄R, -CN, -CONH₂, -Cl, -NC₄H₆O, -NH(CH₂)₃COOH, -NHCOCH₃, -CONHC(CH₃)₃, - CON(CH₃)₂, -CH=CH₂, C1-C18-Alkyl, Hydroxy-C1-C18-Alkyl, -C(=O)O(CH₂CH₂O)ₓR3, - C(=O)NH(CH₂CH₂O)ₓ-R³, -CH2=(CH₂CH₂-O)ₓ-R³ mit x= 1-100 und R³=C1-C30-Alkyl oder der Formel CH₂=CH(OCOR²) mit R²=C1-C18 Alkyl oder der Formel CH₂=CH₂ oder CH₂=CHCH₃ und c) einer mehrfach ungesättigten Komponente, die zur teilweisen Quervernetzung geeignet ist.

Dabei ist es von Vorteil, wenn die Gesamtmenge an einer oder mehreren Acrylatverdickern aus dem Bereich von 0,1 - 8,0 Gew.%, bevorzugt 0,3 - 6 Gew.%, besonders bevorzugt 0,5 - 4 Gew.%, außergewöhnlich bevorzugt 1,5 - 3 Gew.-% gewählt wird, bezogen auf das Gesamtgewicht der Zubereitungen.

Dabei ist es von Vorteil, wenn das Polyvinylpyrrolidon in Gehalten von 0,01 bis 3 Gew.-%, besonders bevorzugt 0,1 bis 1 Gew.-%, außergewöhnlich bevorzugt 0,1 bis 0,9 Gew.-% vorliegt.

Dabei ist es von Vorteil, wenn als kationisches Polymer Polyquatemium-7, Polyquaternium-10 Polyquatemium-44 oder Guar Quats oder eine Kombination aus Polyquats und Guar Quat verwendet wird.

Dabei ist es von Vorteil, wenn das kationische Polymer in Konzentrationen von 0,01 bis 0,6 Gew.%, besonders bevorzugt 0,1 bis 0,3 Gew.%, eingesetzt wird.

Dabei ist es von Vorteil, wenn als kationisches Polymer Polyquaternium-10 und/oder Guar Quat verwendet wird.

Dabei ist es von Vorteil, wenn als kationisches Polymer auschließlich Polyquaternium-10 verwendet wird.

Dabei ist es von Vorteil, wenn zusätzlich ein nichtionisches Tensid enthalten ist, welches einen HLB von 10 bis 20 aufweist.

Als erfindungsgemäß vorteilhaft einzusetzender Acrylatverdicker wird vorteilhaft ein Produkt eingesetzt, dass von der Gesellschaft Noveon unter der Bezeichnung Aqua SF-1 (INCI: Acrylates Copolymer) verkauft wird. Es stellt ein leicht quervernetztes durch Alkalien quellbares Acrylatcopolymer dar, welches drei Strukturkomponenten enthält, nämlich ein oder mehrere Carboxylsäuremonomere mit 3 bis 10 Kohlenstoffatomen, ein oder mehrere Vinylmonomere sowie als dritte Komponente ein oder mehrfach ungesättigte Monomere.

Vorteilhaft sind ferner Verbindungen, die die INCI-Bezeichnung "Acrylates/Ceteth-20 Itaconate Copolymer" (unter der Handelsbezeichnungen Structure 2001® bei der National Starch erhältlich), die die INCI-Bezeichnung "Acrylates Copolymer" (unter der Handelsbezeichnungen SALCARE SC81® bei Ciba erhältlich), die die INCI-Bezeichnung "Acrylates Copolymer" (unter der Handelsbezeichnungen POLYGEL W400® bei 3V erhältlich), die die INCI-Bezeichnung "Acrylates Copolymer" (unter der Handelsbezeichnungen VISCOLAM MAC-7® bei Lamberti erhältlich) und die die INCI-Bezeichnung "Acrylates/ Beheneth-25 Methacrylate Copolymer" (unter der Handelsbezeichnungen TINOVIS GTC® bei Ciba erhältlich) und ähnliche Polymere.

Anionische Tenside im Sinne der Erfindung weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quartemären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:
RNH₂+CH₂CH₂COOH X⁻ (bei pH=2) X⁻ = beliebiges Anion, z.B. **Cl⁻**
RNH₂⁺CH₂CH₂COO⁻ (bei pH=7)
RNHCH₂CH₂COO⁻ B⁺ (bei pH=12) B⁺ = beliebiges Kation, z.B. Na⁺

Typisch für nicht-ionische Tenside sind Polyether-Ketten. Nicht-ionische Tenside bilden in wässrigem Medium keine Ionen.

### A. Anionische Tenside

Erfindungsgemäß vorteilhaft zu verwendende anionische Tenside sind Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate

Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-4 Phosphat,

Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido-MEA-Sulfosuccinat
sowie

Schwefelsäureester, wie
1. Alkylethersulfat, beispielsweise Natrium-, Ammonium-, Magnesium-, MIPA-, TIPA-Laurethsulfat, Natriummyrethsulfat und Natrium C₁₂₋₁₃-Parethsulfat,
2. Alkylsulfate, beispielsweise Natrium-, Ammonium- und TEA-Laurylsulfat.

### B. Amphotere Tenside

Erfindungsgemäß vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

Vorteilhafte nichtionische Tenside mit einem HLB-Wert von 10 bis 20 können beispielsweise sein:

PEG-20 Mandelölglyzerid, PEG-20 Nachtkerzenölglyzerid, PEG-60 hydrogeniertes Rizinusöl, Polysorbat 60, PEG-20 Sorbitan Isostearat, PEG-40 hydrogeniertes Rizinusöl, Polysorbat 80, Succrose Cocoat, PEG-45 Palmkemölglyzeride, PEG-45 Distelölglyzerid, PEG-60 Nachtkerzenölglyzerid, PEG-42 Babassuölglyzerid, PEG-20 Rizinusoleat, Steareth-21, Oleth-20, Polysorbat 40, Laureth-9, Laureth-15, Ceteareth-20, Ceteth-20, PEG-12 Laurat, PEG-24 Stearat, PEG-20 Stearat, PEG-20 Oleat, PEG-75 Lanolin, Laneth-40, PEG-8 Dilaurat, Polysorbat 65, PEG-7 Glyceryl Cocoat, Laneth-10, PEG-12 Oleat, Polyglyzeryl-6 Laurat, Polyglyzeryl-10 Laurat, Polyglyzeryl-10 Myristat, Polyglyzeryl-10 Stearat, Polyglyzeryl-10 Oleat, Polyglyzeryl-10 Isostearat, Polyglyzeryl-10 Diisostearat, PEG-15 Glyzeryloleat, PEG-60 Sorbitan Tetraoleat, PEG-10 Oleylether

Erfindungsgemäß vorteilhafte Filmbildner können dabei aus den in der Tabelle aufgelisteten Verbindungen gewählt werden.

**Tabelle 1: Erfindungsgemäß vorteilhafte Filmbildner**

| **Bezeichnung nach INCI** | **CAS-Nummer** | **Polymertyp** | **Beispiel (Handelsname)** |
|---|---|---|---|
| Polyquatemium-2 | CAS 63451-27-4 | Urea, N, N'- bis[3- (dimethylamino)propyl]- , polymer mit 1, 1'- oxy-bis(2- chloroethan) | Mirapol® A-15 |
| Polyquatemium-5 | CAS 26006-22-4 | Acrylamid, β-Methacryloxyethyltriethylammoniummethosulfat | |
| Polyquaternium-6 | CAS 26062-79-3 | N,N-Dimethyl-N-2-propenyl-2-propen-aminiumchlorid | Merquat® 100 |
| Polyquaternium-7 | CAS 26590-05-6 | N,N-Dimethyl-N-2-propenyl-2-propen-aminiumchlorid, 2-Propenamid | Merquat® S |
| Polyquaternium-10 | CAS 53568-66-4, 55353-19-0, 54351-50-7, 68610-92-4, 81859-24-7 | Quaternäres Ammoniumsalz der Hydroxyethylcellulose | Celquat® SG230M |
| Polyquaternium-11 | CAS 53633-54-8 | Vinylpyrrolidon/dimethylaminoethyl-Methacrylat-Copolymer/Diethylsulfat-Reaktionsprodukt | Gafquat®755N |
| Polyquaternium-16 | CAS 29297-55-0 | Vinylpyrrolidon/vinylimidazolinumm ethochlorid-Copolymer | Luviquat® HM552 |
| Polyquatemium-17 | CAS 90624-75-2 | | Mirapol®AD-1 |
| Polyquaternium-19 | CAS 110736-85-1 | Quatemisierter wasserlöslicher Polyvinylalkohol | |
| Polyquaternium-20 | CAS 110736-86-2 | In Wasser dispergierbarer quatemisierter Polyvinyloctadecylether | |
| Polyquaternium-21 | Polysiloxan- | Polysiloxanpolydimethyl-dimethylammoniumacetat-Copolymer | Abil® B 9905 |
| Polyquaternium-22 | CAS 53694-17-0 | Dimethyldiallylammoniumchlorid/Acrylsäure-Copolymer | Merquat®280 |
| Polyquaternium-24 | CAS 107987-23-5 | Polymeres quaternäres Ammoniumsalz der Hydroxyethylcellulose | Quartisoft® LM-200 |
| Polyquaternium-28 | CAS 131954-48-8 | Vinylpyrrolidon/Methacrylamidoprop yltrimethylammoniumchlorid-Copolymer | Gafquat®HS-100 |
| Polyquaternium-29 | CAS 92091-36-6, 148880-30-2 | Chitosan, das mit Propylenoxid umgesetzt u. mit Epichlorhydrin quaternisiert wurde | Lexquat® CH |
| Polyquaternium-31 | CAS 136505-02-7, 139767-67-7 | Polymeres, quaternäres Ammoniumsalz, das durch die Umsetzung von DMAPA-Acrylate/Acrylsäure/Acrylonitro gens-Copolymeren u. Diethylsulfat hergestellt wird | Hypan® QT 100 |
| Polyquaternium-32 | CAS 35429-19-7 | N,N,N-Trimethyl-2-{[82methyl-1-oxo-2-Propenyl)oxy]-ethanaminiumchlorid, polymer mit 2-Propenamid | |
| Polyquaternium-37 | CAS 26161-33-1 | | |
| Polyquaternium-44 | | Copolymeres quaternes Ammoniumsalz aus Vinylpyrrolidon und quaternisiertem Imidazolin | |

Weitere erfindungsgemäß vorteilhafte Filmbildner stellen Cellulosederivate und quaternisierte Guar Gum Derivate, insbesondere Guar Hydroxypropylammoniumchlorid (z.B. Jaguar Excel®, Jaguar C 162® der Firma Rhodia, CAS 65497-29-2, CAS 39421-75-5) dar.

Auch nichtionische Poly-N-vinylpynrolidoNPolyvinylacetat-Copolymere (z.B. Luviskol VA 64W®, BASF), anionische Acrylat-Copolymere (z.B. Luviflex soft®, BASF), und/oder amphotere Amid/Acrylat/Methacrylat Copolymere (z.B. Amphomer®, National Starch) können erfindungsgemäß vorteilhaft als Filmbildner eingesetzt werden.

Erfindungsgemäß bevorzugte Filmbildner sind Polyquaternium-10, Polyquatemium-22 und Polyquaternium-44 und Jaguar Excel®. Erfindungsgemäß besonders bevorzugt ist Polyquaternium-10 (z.B. Ucare Polymer JR-125® und Ucare Polymer JR-400®, Amerchol) in Kombination mit Jaguar Excel®.

Die erfindungsgemäßen waschaktiven Zubereitungen zeichnen sich in der Regel durch einen Wassergehalt von 95 - 5 Gew.-% aus, bezogen auf das Gesamtgewicht der Zubereitungen und stellen Gele dar.

Vorteilhaft werden die Zubereitungen - sofern es sich um gelförmige Zubereitungen mit Fliessgrenze handelt - so ausgestaltet, dass sie eine Fließgrenze von 0,5 - 20 Pa aufweisen, bevorzugt 1 - 6 Pa.

Als Fließgrenze wird die kritische Schubspannung der Fließkurve angesehen. Sie kann erfindungsgemäß wie folgt ermittelt werden:

Gemessen wird die Fließkurve auf einem schubspannungsgesteuerten Rheometer bei 25°C ± 1°C mit 25 mm Platte/Platte Geometrie bei einem Spalt zwischen 0,8 mm und 1,2 mm, wobei strukturschonend befüllt wird. Es wird eine geeignete konstante Schubspannungszeitrampe vorgegeben und vor dem Test eine entsprechende Strukturerholungszeit eingehalten und die kritische Schubspannung im Maximum der Fließkurve angegeben.

Vorteilhaft werden die Zubereitungen so ausgestaltet, dass sie einen tan δ von 0,05 - 0,6 aufweisen, bevorzugt 0,1 - 0,5.

Unter tan δ wird erfindungsgemäß der Quotient aus dem Verlustmodul und dem Speichermodul verstanden. Der tan δ wird wie folgt ermittelt:

Gemessen werden Verlust- und Speichermodul durch einen dynamischen Frequenztest auf einem schubspannungsgesteuerten Rheometer bei 40 °C ± 1 °C mit 25 mm Platte/Platte Geometrie bei einem Spalt zwischen 0,8 mm und 1,2 mm, wobei strukturschonend befüllt wird. Es wird nach dem Stand der Technik der Frequenztest mit einer entsprechenden Strukturerholungszeit vor dem Test durchgeführt und der tan 8 im Frequenzbereich zwischen 0,05 rad/s und 3,0 rad/s angegeben, bevorzugt zwischen 0,08 rad/s und 1,0 rad/s.

Die Fließgrenze kann durch Erhöhung der Gelbildnerkonzentration angehoben werden. Vorteilhaft im Sinne der Erfindung hat sich der Einsatz von nichtionischen Filmbildnern erwiesen, insbesondere von Polyvinylpyrrolidon (PVP), ganz besonders von PVP, welches unter dem Handelsnamen Luviskol K 30 Pulver (BASF) anbegoten wird. Bevorzugte Einsatzkonzentrationen liegen bei 0,01-3 Gew. %, besonders bevorzugt bei 0,1-1 Gew.%.

Als Perlglanzsysteme können prinzipiell alle gängigen, bekannten Perlglanzrohstoffe verwendet werden. Besonders vorteilhaft ist die Verwendung von PEG-3 Distearat. Ganz besonders vorteilhaft ist es, wenn dieser Perlglanzrohstoff eine Teilchengröße aufweist in der Art, dass 90% der Volumenanteile eine Teilchengröße von kleiner 50µm aufweisen. Die Einsatzkonzentrationen des Perlglanzes liegen vorteilhafterweise bei 5-15 Gew.%.

Anstelle des Perlglanzsystems kann auch ein Trübungsmittel eingesetzt werden. Besonders vorteilhaft ist der Einsatz von Mischungen, bestehend aus Glycol Distearat, Coco-Glucosid, Glyceryl Oleat, Glyceryl Stearate wie sie unter dem Handelsnamen Lamesoft TM Benz (Cognis) angeboten weden.

Vorteilhaft kann auch eine Kombination von Perlglanz und Trübungsmittel eingesetzt werden.

Besonders vorteilhaft bei der Gesichtsreinigung - also nichjt im Falle der Haar- und Kopfhautreinigung - sind Kombinationen von Myrethsulfat mit Cocamidopropylbetain einzusetzen. Besonders vorteilhaft ist Myrethsulfat in Konzentrationen von 1-10 Gew.% und Cocamidopropylbetain in Konzentration von 0.5-10 Gew.% einzusetzen. Ganz besonders vorteilhaft ist es Myrethsulfat in Konzentrationen von 2-6 Gew.% und Cocamidopropylbetain in Konzentrationen von 1-3 Gew. % einzusetzen. Als wichtiges Cotensid können weiterhein 0,5- 5% Alkylpolyglucoside zum Einsatz kommen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüm, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Zubereitungen gemäß der Erfindung sind vorteilhaft auf einen pH-Bereich > 4,2 gepuffert, besonders bevorzugt > 4,5 besonders bevorzugt 4,8-7,5.

Die Herstellung der erfindungsgemäßen kosmetischen und/oder dermatologischen Zubereitungen erfolgt in der dem Fachmann üblichen Weise, zumeist dergestalt, dass die erfindungsgemäß verwendeten grenzflächenaktiven Stoffe bei gleichmäßigem Rühren und gegebenenfalls unter Erwärmen suspendiert und gewünschtenfalls homogenisiert, gegebenenfalls mit weiteren Lipidkomponenten und gegebenenfalls mit einem oder mehreren weiteren Emulgatoren vereinigt, hernach die Ölphase mit der wässrigen Phase, in welche gegebenenfalls ein Verdickungsmittel eingearbeitet worden ist, und welche vorzugsweise etwa die gleiche Temperatur besitzt wie die Ölphase, vermischt, gewünschtenfalls homogenisiert und auf Raumtemperatur abkühlen lässt. Nach Abkühlen auf Raumtemperatur kann, insbesondere, wenn noch flüchtige Bestandteile eingearbeitet werden sollen, nochmaliges Homogenisieren erfolgen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

Aqua SF-1 -sofern eingesetzt - wird mit einem Teil der Wasserphase verdünnt und unter Rühren zur Tensidphase gegeben. Anschließend werden die weiteren Rezepturbestandteile unter Rühren zugegeben und mit NaOH der pH-Wert eingestellt. Bei all dem wird unter möglichst geringer Scherung gerührt.

### Shampoos

| | **1** | **2** | **3** | **4** | **5** | **6** |
|---|---|---|---|---|---|---|
| Natrium Laurylethersulfat | 4 | 4 | 5 | 4 | 6 | 8 |
| Cocamidopropyl Betain | 4 | 3.5 | 3.5 | 3.5 | 4 | 4 |
| Polyquaternium-10 | 0.1 | 0.3 | 0.2 | 0.3 | 0.1 | - |
| Guar Hydroxypropyltrimmoniumchlorid | - | - | - | - | - | 0.1 |
| Acrylates Copolymer (Aqua SF-1) | 3.0 | 1.5 | 2.0 | 2.5 | 2.5 | 2.4 |
| Polyvinylpyrrolidon | 0.5 | 0.5 | 0.4 | 0.5 | 0.6 | 0.5 |
| PEG-40 hydrogeniertes Rizinusöl | 0.2 | 0.6 | 0.2 | 0.8 | 0.6 | 1.2 |
| Perlglanz (PEG-3 Distearat) | 1.5 | 1.5 | - | - | - | - |
| Trübungsmittel Lamesoft TM Benz (Glycol Distearat, Coco-Glucosid, Glyceryl Oleat, Glyceryl Stearate) | - | 4.0 | 3.0 | 4.0 | 5.0 | 4.0 |
| Methylparaben | 0.4 | 0.4 | 0.4 | 0.4 | 0.4 | 0.4 |
| Propylparaben | 0.2 | 0.1 | 0.2 | 0.2 | 0.2 | 0.2 |
| Phenoxyethanol | 0.5 | 0.6 | 0.6 | 0.4 | 0.6 | 0.6 |
| EDTA | 0.3 | 0.2 | 0.2 | 0.1 | 0.2 | 0.2 |
| Natronlauge | q.s. | q.s. | q.s. | q.s. | q.s. | q.s. |
| Citronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

Aqua SF-1 wird mit einem Teil der Wasserphase verdünnt und unter Rühren zur Tensidphase gegeben. Anschließend werden die weiteren Rezepturbestandteile bis auf NaOH und die Schwebekörper unter Rühren zugegeben. Nach erfolgter pH-Einstellung werden die Schwebekörper in die fertige Gelgrundlage unter möglichst geringer Scherung eingerührt.

### Zubereitungen zur Körperreinigung:

| | **1** | **2** | **3** | **4** | **5** | **6** |
|---|---|---|---|---|---|---|
| Natrium Laurethsulfat | 11,0% | 9,5% | 12,0% | 9,0% | 12% | 6% |
| Cocoamidopropylbetain | 4,0% | 4,5% | 1,1% | | | 5% |
| Natrium Cocoamphoacetat | | | | 4,5% | 3,5% | |
| Natriumcocoylglutamat | 1,5% | 2,5% | 0,8% | 0,5% | 1,0% | |
| Acrylates Copolymer (Aqua SF-1) | 3,0% | 2,5% | 2,2% | | | |
| Acrylates/C10-30 Alkyl Acrylat Crosspolymer | | | | | | |
| Magnesium Aluminium Silicat | | | | 3,0% | 2,3% | |
| Polyvinylpyrrolidon | 0,3% | 0,9% | 0,1% | 0,3% | 0,6% | 0,4% |
| Hydroxypropyl Guarhydroxypropyltrimonium Chlorid | | | | 0,1% | 0,1% | |
| PEG-6 Caprylic/Capric Glycerides | | | 1,0% | | | |
| PEG-40 hydriertes Rizinusöl | 0,5% | 0,5% | 0,5% | 0,5% | 0,5% | 1,0% |
| Glycol Distearate | 0,3% | | | | | |
| Glycerin | 0,2% | | | | | 1,5% |
| PEG-7 Glyceryl Cocoat | | 1,5% | | | | 1,0% |
| Styrene/Acrylate Copolymer | | | 1,0% | | | 2,5% |
| PEG-3 Distearat | | | | 2,0% | 2,0% | |
| Trisodium EDTA | | 0,2% | | | 0,2% | |
| Benzophenone-4 | | 0,1% | | | 0,1% | |
| Polyethylene | 1,5% | | | 5% | 2% | |
| Pigmente | q.s. | q.s. | q.s. | q.s. | q.s. | |
| Polyquaternium-10 | | 0,2% | | | | 0,1% |
| Polyquaternium-7 | | | 0,4% | 0,2% | | 0,2% |
| Konservierungsstoffe | q.s. | q.s. | q.s. | q.s. | q.s. | q.s. |
| Farbstoff | q.s. | q.s. | q.s. | q.s. | q.s. | q.s. |
| Citronensäure | q.s. | q.s. | q.s. | q.s. | q.s. | q.s. |
| NaOH | q.s. | q.s. | q.s. | q.s. | q.s. | q.s. |
| Parfum | q.s. | q.s. | q.s. | q.s. | q.s. | q.s. |
| Wasser | Ad 100 | ad 100 | ad 100 | ad 100 | ad 100 | ad 100 |

### Zubereitungen zur Gesichtsreinigung:

| | 1 | 2 | 3 | 4 | 5 |
|---|---|---|---|---|---|
| Natrium Myrethsulfat | 2% | -- | 3% | 5% | 2% |
| Natrium Laurethsulfat | -- | 2% | 1% | -- | 2% |
| Cocoamidopropylbetain | 1% | 2% | 1% | 1% | 1% |
| Acrylates Copolymer (Aqua SF-1) | 0,3% | 0,5% | 0,2% | 0,2% | 1% |
| Polyvinylpyrrolidon | 0,2% | 0,5% | 0,3% | 0,2% | 0,1% |
| Natriumhydroxid | 0,5% | 0,5% | 0,5% | 0,5% | 0,5% |
| PEG-7 Glyceryl Cocoat | 1% | 0,5% | -- | -- | -- |
| Na3HEDTA | 0,5% | 0,5% | 0,5% | 0,5% | 0,5% |
| Polyquaternium-10 | 0,1% | -- | 0,2% | -- | -- |
| Licochalcone A | 0.025 % | --- | 0.05% | --- | 0.02% |
| Ubiquinon | -- | 0.01% | -- | --- | 0,025 % |
| Polyethylene | 1% | 0,5% | 0,1% | - | - |
| PEG-40 hydriertes Rizinusöl | -- | 0,5% | -- | 1% | -- |
| Phenoxyethanol | 0,5% | 0,5% | 0,5% | 0,5% | 0,5% |
| Parabene | 0,2% | 0,2% | 0,2% | 0,2% | 0,2% |
| Parfum | q.s. | q.s. | q.s. | q.s. | q.s. |
| Wasser | ad 100 | ad 100 | ad 100 | ad 100 | ad 100 |

## Patentansprüche

1. Kosmetische und dermatologische waschaktive Zubereitungen zur Reinigung der Haut und/oder zur Haar- und Kopfhautreinigung, enthaltend
(a) eine wirksame Menge an einem oder mehreren anionischen Tensiden,
(b) eine wirksame Menge an einem oder mehreren amphotheren Tensiden,
(c) eine wirksame Menge eines oder mehrerer gelbildender Acrylatverdicker gewählt aus der Gruppe der vernetzten alkali-quellbaren Acrylat Copolymere,
(d) ein Homo- oder Copolymer auf der Basis von Vinylpyrrolidon, besonders bevorzugt Polyvinylpyrrolidon,
(e) gewünschtenfalls weitere übliche Hilfs- und/oder Zusatzstoffe, insbesondere Wasser.

2. Zubereitungen nach Anspruch 1, **dadurch gekennzeichnet, dass** die Formulierungen zusätzlich kationische Polymere enthalten.

3. Zubereitungen nach einem der vorhergehenden Ansprüche, **dadurch gekennzeichnet, dass** als anionisches Tensid Laurylethersulfat und/oder Myrethethersulfate und als amphotheres Tensid Cocoamidopropylbetain gewählt wird.

4. Zubereitungen nach einem der vorangehenden Patentansprüche, dadurch gekennzichnet, dass die Gesamtmenge an einer oder mehreren erfindungsgemäß verwendeten Tenside aus dem Bereich von 0,1 -25 Gew.-%, bevorzugt 1-12 Gew.-%, insbesondere bevorzugt 2-10 Gew.-% gewählt wird, jeweils bezogen auf das Gesamtgewicht der Zubereitungen.

5. Zubereitungen nach einem der vorangehenden Patentansprüche, **dadurch gekennzeichnet, dass** die Gesamtmenge an einer oder mehreren erfindungsgemäß verwendeten anionischen Tenside aus dem Bereich von 5 bis 15 Gew.-%, bevorzugt 6 bis 12 Gew.-% gewählt wird, jeweils bezogen auf das Gesamtgewicht der Zubereitungen.

6. Zubereitungen nach einem der vorangehenden Patentansprüche, **dadurch gekennzeichnet, dass** die Gesamtmenge an einer oder mehreren erfindungsgemäß verwendeten amphotheren Tenside aus dem Bereich von 0,5 bis 10 Gew.-%, bevorzugt 1 bis 5 Gew.-% gewählt wird, jeweils bezogen auf das Gesamtgewicht der Zubereitungen.

7. Zubereitungen nach Anspruch 1, **dadurch gekennzeichnet, dass** als Acrylatverdicker ein Copolymer verwendet wird, bestehend aus a) einem Acrylatmonomer ausgewählt aus Acrylsäure, Methacrylsäure, Itaconsäure, Fumarsäure, Crotonsäure, Aconitsäure oder Maleinsäure, b) einem a,b-ethylenisch ungesättigten Monomer der allgemeinen Formel CH₂=CXY mit X=H, CH₃, -C1-C30-Alkyl, -CH₂-C(=O)O(CH₂-CH₂-O)ₓ-R³, -CH₂-C(=O)NH(CH₂-CH₂-O)ₓ-R³, -CH₂-CH₂=(CH₂-CH₂-O)ₓ-R³ mit x= 1-100 und R³=C1-C30 Alkyl oder Cl und Y= -COOR, -C₆H₄R, -CN, - CONH₂, -Cl, -NC₄H₆O, -NH(CH₂)₃COOH, -NHCOCH₃, -CONHC(CH₃)₃, - CON(CH₃)₂, -CH=CH₂, C1-C18-Alkyl, Hydroxy-C1-C18-Alkyl, -C(=O)O(CH₂-CH₂-O)ₓ-R3, -C(=O)NH(CH₂-CH₂-O)ₓ-R³, -CH2=(CH₂-CH₂-O)ₓ-R³ mit x= 1-100 und R³=C1-C30-Alkyl oder der Formel CH₂=CH(OCOR²) mit R²=C1-C18 Alkyl oder der Formel CH₂=CH₂ oder CH₂=CHCH₃ und c) einer mehrfach ungesättigten Komponente, die zur teilweisen Quervernetzung geeignet ist.

8. Zubereitungen nach Anspruch 1, **dadurch gekennzeichnet, dass** die Gesamtmenge an einer oder mehreren Acrylatverdickern aus dem Bereich von 0,1 - 8,0 Gew.-%, bevorzugt 0,3 - 6 Gew.-%, besonders bevorzugt 0,5 - 4 Gew.-%, außergewöhnlich bevorzugt 1,5-3 Gew.-% gewählt wird, bezogen auf das Gesamtgewicht der Zubereitungen.

9. Zubereitungen nach Anspruch 1, **dadurch gekennzeichnet, dass** das Polyvinylpyrrolidon in Gehalten von 0,01 bis 3 Gew.-%, besonders bevorzugt 0,1 bis 1 Gew.-%, außergewöhnlich bevorzugt 0,1 bis 0,9 Gew.-% vorliegt.

10. Zubereitungen nach Patentanspruch 2, **dadurch gekennzeichnet, dass** als kationisches Polymer Polyquaternium-7, Polyquaternium-10 Polyquaternium-44 oder Guar Quats oder eine Kombination aus Polyquats und Guar Quat verwendet wird.

11. Zubereitungen nach Patentanspruch 2 oder 10, **dadurch gekennzeichnet, dass** das kationische Polymer in Konzentrationen von 0,01 bis 0,6 Gew.%, besonders bevorzugt 0,1 bis 0,3 Gew.%, eingesetzt wird.

12. Zubereitungen zur Haar- und Kopfhautreinigung nach Patentanspruch 2 oder 11, **dadurch gekennzeichnet, dass** als kationisches Polymer Polyquaternium-10 und/oder Guar Quat verwendet wird.

13. Zubereitungen nach Patentanspruch 12, **dadurch gekennzeichnet, dass** als kationisches Polymer auschließlich Polyquaternium-10 verwendet wird.

14. Zubereitung nach einem der vorhergehenden Ansprüche, **dadurch gekennzeichnet, dass** zusätzlich ein nichtionisches Tensid enthalten ist, welches einen HLB von 10 bis 20 aufweist.

## Claims

1. Cosmetic and dermatological washing-active preparations for cleaning the skin and/or for hair and scalp cleaning, comprising
(a) an effective amount of one or more anionic surfactants,
(b) an effective amount of one or more amphoteric surfactants,
(c) an effective amount of one or more gel-forming acrylate thickeners chosen from the group of crosslinked alkali-swellable acrylate copolymers,
(d) a homopolymer or copolymer based on vinylpyrrolidone, particularly preferably polyvinylpyrrolidone,
(e) if desired further customary auxiliaries and/or additives, in particular water.

2. Preparations according to Claim 1, **characterized in that** the formulations additionally comprise cationic polymers.

3. Preparations according to one of the preceding claims, **characterized in that** the anionic surfactant chosen is lauryl ether sulfate and/or myreth ether sulfate and the amphoteric surfactant chosen is cocoamidopropylbetaine.

4. Preparations according to one of the preceding patent claims, **characterized in that** the total amount of one or more surfactants used according to the invention is chosen from the range from 0.1-25% by weight, preferably 1-12% by weight, particularly preferably 2-10% by weight, in each case based on the total weight of the preparations.

5. Preparations according to one of the preceding patent claims, **characterized in that** the total amount of one or more anionic surfactants used according to the invention is chosen from the range from 5 to 15% by weight, preferably 6 to 12% by weight, in each case based on the total weight of the preparations.

6. Preparations according to one of the preceding patent claims, **characterized in that** the total amount of one or more amphoteric surfactants used according to the invention is chosen from the range from 0.5 to 10% by weight, preferably 1 to 5% by weight, in each case based on the total weight of the preparations.

7. Preparations according to Claim 1, **characterized in that** the acrylate thickener used is a copolymer consisting of a) an acrylate monomer chosen from acrylic acid, methacrylic acid, itaconic acid, fumaric acid, crotonic acid, aconitic acid or maleic acid, b) an α,β-ethylenically unsaturated monomer of the general formula CH₂-CXY where X=H, CH₃, -C1-C30-alkyl, -CH₂-C(=O)O(CH₂-CH₂-O)ₓ-R³, -CH₂-C(=O)NH(CH₂-CH₂-O)ₓ-R³, -CH₂CH₂=(CH₂-CH₂-O)ₓ-R³ where x = 1-100 and R³ = C1-C30 alkyl or Cl and Y = -COOR, -C₆H₄R, -CN, -CONH₂, -Cl, -NC₄H₆O, -NH (CH₂)₃COOH, -NHCOCH₃, -CONHC (CH₃)₃, -CON (CH₃)₂, -CH=CH₂, C1-C18-alkyl, hydraxy-C1-C18-alkyl, -C (=O) O(CH₂-CH₂-O)ₓ-R³, -C(=O)NH (CH₂-CH₂)ₓ-R³, -CH₂= (CH₂-CH-O)ₓ-R³ where x = 1-100 and R³=C1-C30-alkyl or of the formula CH₂=CH(OCOR²) where R²=C1-C18 alkyl or of the formula CH₂=CH₂ or CH₂=CHCH₃ and c) a polyunsaturated component which is suitable for partial crosslinking.

8. Preparations according to Claim 1, **characterized in that** the total amount of one or more acrylate thickeners is chosen from the range 0.1-8.0% by weight, preferably 0.3-6% by weight, particularly preferably 0.5-4% by weight, extraordinarily preferably 1.5-3% by weight, based on the total weight of the preparations.

9. Preparations according to Claim 1, **characterized in that** the polyvinylpyrrolidone is present in contents of from 0.01 to 3% by weight, particularly preferably 0.1 to 1% by weight, extraordinarily preferably 0.1 to 0.9% by weight.

10. Preparations according to Patent Claim 2, **characterized in that** the cationic polymer used is polyquaternium-7, polyquaternium-10, polyquaternium-44 or guar quats or a combination of polyquats and guar quat.

11. Preparations according to Patent Claim 2 or 10, **characterized in that** the cationic polymer is used in concentrations of from 0.01 to 0.6% by weight, particularly preferably 0.1 to 0.3% by weight.

12. Preparation for hair and scalp cleaning according to Patent Claim 2 or 11, **characterized in that** the cationic polymer used is polyquaternium-10 and/or guar quat.

13. Preparation according to Patent Claim 12, **characterized in that** the cationic polymer used is exclusively polyquaternium-10.

14. Preparation according to one of the preceding claims, **characterized in that** a nonionic surfactant is additionally present which has an HLB of from 10 to 20.

## Revendications

1. Préparations lavantes cosmétiques et dermatologiques destinées au nettoyage de la peau et/ou au nettoyage des cheveux et du cuir chevelu, contenant
(a) une quantité efficace d'un ou plusieurs tensioactifs anioniques,
(b) une quantité efficace d'un ou plusieurs tensioactifs amphotères,
(c) une quantité efficace d'un ou plusieurs épaississants acrylate gélifiants choisis dans le groupe des copolymères d'acrylate réticulés susceptibles de gonfleur en présence de solutions alcalines,
(d) un homopolymère ou copolymère à base de vinylpyrrolidone, de façon particulièrement préférée de polyvinylpyrrolidone,
(e) si on le désire d'autres additifs et/ou adjuvants usuels, l'eau en particulier.

2. Préparations selon la revendication 1, **caractérisées en ce que** les compositions contiennent en plus des polymères cationiques.

3. Préparations selon l'une quelconque des revendications précédentes, **caractérisées en ce qu'**on choisit comme tensioactif anionique le lauryléthersulfate et/ou des myrethéthersulfates et comme tensioactif amphotère la cocosamidopropylbétaïne.

4. Préparations selon l'une quelconque des revendications précédentes, **caractérisées en ce que** la quantité totale d'un ou plusieurs tensioactifs utilisés selon l'invention est choisie dans la plage de 0,1 - 25 % en poids, de préférence 1 - 12 % en poids, de façon particulièrement préférée 2 - 10 % en poids, chaque fois par rapport au poids total des préparations.

5. Préparations selon l'une quelconque des revendications précédentes, **caractérisées en ce que** la quantité totale d'un ou plusieurs tensioactifs anioniques utilisés selon l'invention est choisie dans la plage de 5 à 15 % en poids, de préférence de 6 à 12 % en poids, chaque fois par rapport au poids total des préparations.

6. Préparations selon l'une quelconque des revendications précédentes, **caractérisées en ce que** la quantité totale d'un ou plusieurs tensioactifs amphotères utilisés selon l'invention est choisie dans la plage de 0,5 à 10 % en poids, de préférence de 1 à 5 % en poids, chaque fois par rapport au poids total des préparations.

7. Préparations selon la revendication 1, **caractérisées en ce qu'**on utilise comme épaississant acrylate un copolymère constitué de a) un monomère acrylate choisi parmi l'acide acrylique, l'acide méthacrylique, l'acide itaconique, l'acide fumarique, l'acide crotonique, l'acide aconitique ou l'acide maléique, b) un monomère à insaturation α,β-éthylénique de formule générale CH₂=CXY où X = H, CH₃, -alkyle en C₁-C₃₀, -CH₂-C(=O)O(CH₂-CH₂-O)ₓ-R³,
-CH₂-C(=O)NH(CH₂-CH₂-O)ₓ-R³, -CH₂-CH₂=(CH₂-CH₂-O)ₓ-R³ où x = 1-100 et R³ = alkyle en C₁-C₃₀ ou Cl et Y = -COOR, -C₆H₄R, -CN, -CONH₂, -Cl, -NC₄H₆O, -NH (CH₂)₃COOH, -NHCOCH₃, -CONHC(CH₃)₃, -CON(CH₃)₂, -CH=CH₂, alkyle en C₁-C₁₈, hydroxyalkyle(C₁-C₁₈), -C (=O)O(CH₂-CH₂-O)-R³, -C(=O)NH(CH₂-CH₂-O)ₓ-R³, -CH₂=(CH₂-CH₂-O)ₓ-R³ où x = 1-100 et R³ = alkyle en C₁-C₃₀ ou de formule CH₂=CH (OCOR²) où R² = alkyle en C₁-C₁₈ ou de formule CH₂=CH₂ ou CH₂=CHCH₃ et c) est un composant polyinsaturé qui est approprié à la réticulation transversale partielle.

8. Préparations selon la revendication 1, **caractérisées en ce que** la quantité totale d'un ou plusieurs épaississants acrylate est choisie dans la plage de 0,1 - 8,0 % en poids, de préférence 0,3 - 6 % en poids, de façon particulièrement préférée 0,5 - 4 % en poids, de façon tout particulièrement préférée 1,5 - 3 % en poids, par rapport au poids total des préparations.

9. Préparations selon la revendication 1, **caractérisées en ce que** la polyvinylpyrrolidone est présente en proportions de 0,01 à 3 % en poids, de façon particulièrement préférée 0,1 à 1 % en poids, de façon tout particulièrement préférée 0,1 à 0,9 % en poids.

10. Préparations selon la revendication 2, **caractérisées en ce qu'**on utilise comme polymère cationique le polyquaternium-7, le polyquaternium-10, le polyquaternium-44 ou des guar quats ou une association de polyquats et guar quat.

11. Préparations selon la revendication 2 ou 10, **caractérisées en ce qu'**on utilise le polymère cationique à des concentrations de 0,01 à 0,6 % en poids, de façon particulièrement préférée 0,1 à 0,3 % en poids.

12. Préparations destinées au nettoyage des cheveux et du cuir chevelu selon la revendication 2 ou 11, **caractérisées en ce qu'**on utilise comme polymère cationique du polyquaternium-10 et/ou guar quat.

13. Préparations selon la revendication 12, **caractérisées en ce qu'**on utilise comme polymère cationique exclusivement du polyquaternium-10.

14. Préparation selon l'une quelconque des revendications précédentes, **caractérisée en ce qu'**en plus est contenu un tensioactif non ionique qui présente un rapport HLB de 10 à 20.
